# EUROPÄISCHE PATENTANMELDUNG

(11) **EP 2 762 009 A1**
(43) Veröffentlichungstag der Anmeldung: **06.08.2014**
(21) Anmeldenummer: 13153977.7
(22) Anmeldetag: 05.02.2013
(51) Int. Cl.: A23K 1/16, A23K 1/18, C11B 1/00, C12N 1/06

(54) **Verbesserung der Bioverfügbarkeit von Wertstoffen aus Mikroorganismen**

(71) Anmelder: Evonik Industries AG, 45128 Essen (DE)
(72) Erfinder: Rudinger, Nicolas, 40215 Düsseldorf (DE); Rabe, Christian, 63768 Hösbach (DE)

(57) **Zusammenfassung**

Erfindungsgemäß wurde gefunden, dass ein sehr schonender Aufschluss Wertstoff-haltiger Zellen möglich ist, sofern die Zellen vor Durchführung des Zellaufschlusses aufgequollen werden.

## Beschreibung

Die vorliegende Erfindung betrifft ein Verfahren zum schonenden Aufschluss von Wertstoffe enthaltenden Zellen, Nahrungsmittel und Futtermittel, die solche durch schonenden Aufschluss erhaltene Wertstoffe enthalten, sowie Tiere, die durch Fütterung mit solchen Futtermitteln erhalten wurden.

Die Bedeutung von mikrobiellen Zellen zur Herstellung von Wertstoffen ist dem Fachmann bekannt. Ein Beispiel für solche Wertstoffe stellen Nahrungsmittelkomponenten dar, insbesondere Lipide wie etwa polyungesättigte Fettsäuren. Für die Herstellung von solchen Wertstoffen spielen neben Bakterien und Hefen insbesondere auch weitere Pilze sowie Algen eine besondere Rolle.

Bestimmte Wertstoffe, insbesondere polyungesättigte Fettsäuren (PUFAs), stellen eine wichtige Komponente für die Ernährung von Mensch und Tier dar. Als Quelle für omega-3-Fettsäuren wurde anfangs vor allem Fisch verwendet. Später wurde entdeckt, dass bestimmte Mikroben heterotroph omega-3-Fettsäuren in großen Mengen produzieren, wobei die Fettsäureproduktion durch Wahl spezifischer Reaktionsparameter vorteilhaft beeinflusst werden kann. Die omega-3-Fettsäuren können anschließend aus den Zellen gewonnen werden oder aber die Zellen in Form von Biomasse direkt in Futter- oder Nahrungsmitteln eingesetzt werden.

Ein Problem bei der Verwendung und Verarbeitung von zahlreichen Wertstoffen, insbesondere von polyungesättigten Fettsäuren, stellt ihre Instabilität gegenüber oxidativen Abbau dar: Sobald der Wertstoff aus den Zellen isoliert wird, ist die Gefahr des oxidativen Abbaus stark erhöht, da der Schutz durch die umgebende Zellmembran nicht mehr gegeben ist. Wenn der Wertstoff jedoch nicht aus den Zellen isoliert wird, ist die Bioverfügbarkeit reduziert, d.h. der Wertstoff kann von dem konsumierenden Tier nicht verwertet werden, da die Zellwand nicht oder nicht in ausreichender Menge gespalten werden kann. Ein Kompromiss besteht entsprechend darin, den Wertstoff erst unmittelbar vor seinem Einsatz bzw. erst bei Herstellung des Futter- oder Nahrungsmittels aus den Zellen freizusetzen. Auf diese Weise wird sichergestellt, dass der Wertstoff auf bestmögliche Weise stabilisiert wird, bevor er seiner Anwendung zugeführt wird. In diesem Sinne wird in US 2012/0183668 A1 beschrieben, dass zur Herstellung von Futtermitteln PUFAs enthaltende Zellen mit anderen Futtermittelinhaltsstoffen vermischt werden und das so erhaltene Gemisch zwecks Herstellung eines Futtermittels anschließend einem Extrusionsverfahren unterworfen wird.

Es hat sich jedoch herausgestellt, dass bei Einsatz der PUFAs enthaltenden Zellen der Zellaufschluss in der Regel nur unzureichend erfolgt bzw. dass zur Erzielung eines zufriedenstellenden Zellaufschlusses sehr harsche Zellaufschlussbedingungen in Form eines hohen mechanischen Energieeintrags gewählt werden müssen, wobei selbst dann nicht sichergestellt ist, dass der Zellaufschluss tatsächlich zufriedenstellend verläuft. Bei dem anzuwendenden hohen Energieeintrag kann es im Übrigen zur Schädigung der PUFAs oder anderer Bestandteile der Futtermittelmischung kommen.

Die Aufgabe der vorliegenden Aufgabe bestand daher darin, ein schonenderes Verfahren zur Herstellung von Futtermitteln, die Wertstoffe, vorzugsweise Lipide, insbesondere polyungesättigte Fettsäuren, enthalten, zur Verfügung zu stellen, wobei eine Schädigung der Wertstoffe bestmöglich vermieden werden sollte.

Überraschenderweise wurde erfindungsgemäß gefunden, dass der mechanische Energieeintrag deutlich reduziert werden kann, wenn vor Durchführung des Zellaufschlussverfahrens die Zellen durch Zuführung von Wasser oder einer wässrigen Lösung aufgequollen werden.

Ein erster Gegenstand der vorliegenden Erfindung ist daher ein Verfahren zum Aufschluss von Zellen, die Wertstoffe, insbesondere Lipide, vorzugsweise ungesättigte Fettsäuren, enthalten, **dadurch gekennzeichnet, dass** die Zellen vor dem Zellaufschluss aufgequollen werden.

Unter "Aufquellen der Zellen" ist erfindungsgemäß zu verstehen, dass Wasser (oder eine wässrige Lösung) in die Zellen eindringt und hierdurch der osmotische Druck in den Zellen erhöht wird.

Das Aufquellen der Zellen erfolgt erfindungsgemäß vorzugsweise dadurch, dass ausgehend von einer Zellmasse mit einem Feuchtigkeitsgehalt von weniger als 15 Gew.-%, insbesondere 1 - 14 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%, eine Zellsuspension mit einem Feuchtigkeitsgehalt von mindestens 30 Gew.-%, vorzugsweise 30 bis 90 Gew.-%, besonders bevorzugt 40 bis 80 Gew.-%, insbesondere 50 bis 75 Gew.-%, hergestellt wird.

Der Zellaufschluss kann unter Einsatz der dem Fachmann bekannten Zellaufschlussverfahren erfolgen, wie beispielsweise durch einen Schneckenextruder, eine Schlägermühle, eine Luftdüsenmühle oder durch Anwendung erhöhten Drucks, beispielsweise durch das sogenannte French-Press-Verfahren.

Der Zellaufschluss wird erfindungsgemäß vorzugsweise unter Verwendung eines Rotor-Stator-Systems durchgeführt. Das Rotor-Stator-System basiert auf einem stationären Teil, der Stator genannt wird, und einem rotierenden Teil, dem Rotor. Der Rotor besitzt typischerweise eine Umfangsgeschwindigkeit von mindestens 5 m/s, beispielsweise 10 bis 30 m/s, die Spaltbreite zwischen Rotor und Stator kann beispielsweise 0,1 - 0,5 mm betragen. Zur Durchführung des Zellaufschlusses wird die Zell-Suspension in den Zwischenraum zwischen Stator und Rotor zugegeben. In diesem Spalt erfahren die Zellen eine Scherbeanspruchung und zusätzlich entstehen Turbulenzen. Diese beiden Faktoren bewirken den Zellaufschluss.

Der Energieeintrag auf die Zellen, insbesondere unter Verwendung eines Rotor-Stator-Systems, beträgt erfindungsgemäß vorzugsweise maximal 50 kWh pro Tonne Suspension, insbesondere maximal 40, 35 oder 30 kWh pro Tonne Suspension, besonders bevorzugt maximal 25, 20 oder 15 kWh pro Tonne Suspension. Bevorzugte Bereiche stellen hierbei Energieeinträge von 0,1 - 50 kWh pro Tonne Suspension, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, dar.

Die "Zellaufschlussrate" des erfindungsgemäßen Verfahrens beträgt vorzugsweise mindestens 50 %, besonders bevorzugt mindestens 60, 70 oder 80 %, vor allem mindestens 85, 90 oder 95 %. Unter der "Zellaufschlussrate" ist die Anzahl der aufgeschlossenen Zellen nach Beendigung des Zellaufschlussverfahrens im Verhältnis zur Gesamtzahl der Zellen zu verstehen. Die Zellaufschlussrate kann beispielsweise visuell unter Verwendung eines Mikroskops bestimmt werden als das Verhältnis der Anzahl aufgeschlossener Zellen in Bezug zur Gesamtzahl der Zellen.

Um die Wertstoffe, insbesondere Lipide, gegen oxidativen Abbau zu stabilisieren, können in der Zellsuspension, die für den Zellaufschluss verwendet wird, zusätzlich Antioxidantien enthalten sein. Bevorzugte Antioxidantien stellen hierbei BHT, BHA, TBHA, Ethoxychin, beta-Carotin, Vitamin E und Vitamin C dar. Das Antioxidans ist, sofern eingesetzt, vorzugsweise in einer Menge von 0,01 bis 2 Gew.-% enthalten.

Um den Zellaufschluss zu erleichtern können gegebenenfalls auch geringe Enzymmengen enthalten sein, die zum Verdau der Zellwand beitragen und dadurch die Freisetzung der Lipide erleichtern. Das Zellwand spaltende Enzym ist, sofern eingesetzt, vorzugsweise in einer Menge von 0,01 bis 0,5 Gew.-% in der Zellsuspension enthalten.

In einer erfindungsgemäß bevorzugten Ausführungsform wird der Zellaufschluss jedoch in Abwesenheit von Enzymen, insbesondere in Abwesenheit von Enzymen, die zum Verdau der Zellwand beitragen, durchgeführt. Denn das Enzym müsste nach seinem Einsatz wieder inaktiviert werden. Außerdem wäre eine größere Wassermenge als erfindungsgemäß bevorzugt erforderlich, um das Enzym effektiv einzusetzen.

Bei den erfindungsgemäß im Zellaufschlussverfahren einzusetzenden Zellen kann es sich um Zellen handeln, die bereits natürlicherweise Wertstoffe, vorzugsweise Lipide, insbesondere PUFAs, produzieren, es kann sich jedoch auch um Zellen handeln, die durch entsprechende gentechnische Verfahren dazu in die Lage versetzt wurden, Lipide, insbesondere PUFAs, zu produzieren. Die Produktion kann hierbei autotroph, mixotroph oder heterotroph erfolgen.

Bevorzugt werden erfindungsgemäß Zellen eingesetzt, die Lipide, insbesondere PUFAs, heterotroph produzieren. Es handelt sich bei den Zellen erfindungsgemäß vorzugsweise um Algen, Pilze, insbesondere Hefen, oder Protisten, es kommen jedoch etwa auch Zellen von öl-produzierenden Pflanzen in Betracht. Besonders bevorzugt handelt es sich um Zellen mikrobieller Algen oder Pilze.

Als Zellen von öl-produzierenden Pflanzen kommen insbesondere die Samen von Soja, Flachs, Raps, Mais, Baumwolle, Distel und Sonnenblume in Betracht.

Als Zellen von öl-produzierenden Hefen kommen insbesondere Stämme von Yarrowia, Candida, Rhodotorula, Rhodosporidium, Cryptococcus, Trichosporon und Lipomyces in Betracht.

Erfindungsgemäß werden vorzugsweise Zellen des Taxons Labyrinthulomycetes (Labyrinthulea, Netzschleimpilze, Schleimnetze) eingesetzt, insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium und Ulkenia. Besonders bevorzugt werden erfindungsgemäß Zellen der Gattungen Thraustochytrium, Schizochytrium und Ulkenia eingesetzt, vor allem solche der Gattung Thraustochytrium.

Die erfindungsgemäß einzusetzenden Zellen werden vorzugsweise in Form von sogenannter "Biomasse" eingesetzt. Bei der Biomasse handelt es sich vorzugsweise um das Produkt eines fermentativen Kultivierungsverfahrens. Die Biomasse kann entsprechend neben den aufzuschließenden Zellen auch noch Bestandteile des Fermentationsmediums enthalten. Bei diesen Bestandteilen kann es sich insbesondere um Salze, Antischaummittel und nicht umgesetzte Kohlenstoffquelle und/oder Stickstoffquelle handeln. Der Zellgehalt in dieser Biomasse beträgt vorzugsweise mindestens 70 Gew.-%, vorzugsweise mindestens 75 Gew.- %. Der Zellgehalt in der Biomasse kann gegebenenfalls vor Durchführung des Zellaufschlussverfahrens durch entsprechende Waschschritte beispielsweise auf mindestens 80 oder mindestens 90 Gew.-% erhöht werden. Die erhaltene Biomasse kann jedoch auch direkt in dem Zellaufschlussverfahren eingesetzt werden.

Die erfindungsgemäß im Zellaufschlussverfahren einzusetzenden Zellen zeichnen sich vorzugsweise dadurch aus, dass sie einen Wertstoff-Gehalt, vorzugsweise Lipid-Gehalt, besonders bevorzugt PUFA-Gehalt, von mindestens 20 Gew.-%, vorzugsweise mindestens 30 Gew.-%, insbesondere mindestens 40 Gew.-%, jeweils bezogen auf die Zelltrockenmasse, aufweisen.

In einer bevorzugten Ausführungsform liegt ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Weiterhin umfassen die in der Zelle enthaltenen Lipide vorzugsweise polyungesättigte Fettsäuren (PUFAs), wobei vorzugsweise mindestens 10 Gew.-%, insbesondere mindestens 20 Gew.-%, besonders bevorzugt 20 bis 60 Gew.%, insbesondere 20 bis 40 Gew.-%, der in der Zelle enthaltenen Fettsäuren PUFAs darstellen.

Unter polyungesättigten Fettsäuren (PUFAs) werden erfindungsgemäß Fettsäuren verstanden, die mindestens zwei C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5*Z*,8*Z*,11*Z*,14*Z*,17*Z*)-Eicosa-5,8,11,14,17-pentaensäure, und die Docosahexaensäure (DHA, 22:6ω-3), insbesondere die (4*Z*,7*Z*,10*Z*,13*Z*,16*Z*,19*Z*)-Docosa-4,7,10,13,16,19-hexaensäure, dar, wobei die Docosahexaensäure besonders bevorzugt ist.

Verfahren zur Herstellung der Lipide, insbesondere PUFAs, enthaltenden Zellen insbesondere der Ordnung Thraustochytriales sind im Stand der Technik ausführlich beschrieben. Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden (WO 01/54510). Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon (WO 01/54510).

Die Fermentation der Zellen erfolgt vorzugsweise in einem Medium mit niedriger Salinität, isnbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Die Fermentation der Zellen erfolgt vorzugsweise bei einem pH-Wert von 5 bis 11, insbesondere 6 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Nach Beendigung der Fermentation erfolgt die Ernte der Zellen. Durch Zentrifugation, Filtration, Dekantieren oder Lösungsmittelverdampfung kann ein Großteil des Fermentationsmediums von der Biomasse abgetrennt werden. Die Lösungsmittelverdampfung erfolgt vorzugsweise unter Einsatz eines Trommeltrockners, eines Tunneltrockners, durch Sprühtrocknung oder Vakuumverdampfung. Anschließend wird die erhaltene Biomasse gegebenenfalls weiter getrocknet, vorzugsweise durch Fließbettgranulation. Vorzugsweise wird durch das Trocknen der Feuchtigkeitsgehalt auf unter 15 Gew.-%, insbesondere auf unter 10 Gew.-%, besonders bevorzugt auf unter 5 Gew.-% reduziert.

Vorzugsweise erfolgt nach der Ernte der Zellen oder gegebenenfalls auch schon kurz vor der Ernte der Zellen eine Pasteurisierung der Zellen, um die Zellen abzutöten und Enzyme, die den Abbau der Lipide befördern könnten, zu inaktivieren.

Die auf diese Weise erhaltene - vorzugsweise getrocknete - Biomasse kann nun eingesetzt werden, um die Zellsuspension für den Zellaufschluss durch das Rotor-Stator-System herzustellen. Zur Herstellung der Suspension wird Wasser oder eine wässrige Lösung eingesetzt. Die Suspension kann direkt im Rotor-Stator-System hergestellt werden, so dass Herstellung der Suspension und Zellaufschluss in einem Schritt erfolgen. Alternativ wird zunächst in einem Rührsystem die Zellsuspension hergestellt und diese anschließend zwecks Zellaufschluss in dem Rotor-Stator-System eingesetzt.

In einer bevorzugten Ausführungsform erfolgt die Herstellung der Suspension im Rotor-Stator-System unter Verwendung eines feststoffmischenden Aufsatzes. Unter einem feststoffmischenden Aufsatz ist hierbei eine Vorrichtung zu verstehen, die das separate Einbringen von einerseits Feststoff und andererseits Wasser bzw. wässriger Lösung in das Rotor-Stator-System erlaubt. Die Suspension wird somit erst während des Zellaufschlusses bzw. unmittelbar vor dem Zellaufschluss durch Vermischung in dem feststoffmischenden Aufsatz hergestellt. Erfindungsgemäß wurde gefunden, dass unter Verwendung eines solchen feststoffmischenden Aufsatzes Suspensionen mit sehr hohen Feststoffgehalten dem Zellaufschluss unterzogen werden können, was mit Hinblick auf die nachfolgende Verarbeitung besonders vorteilhaft ist. Suspensionen, die unter Verwendung eines feststoffmischenden Aufsatzes in dem Rotor-Stator-System eingesetzt werden, weisen vorzugsweise einen Feststoffgehalt von 20-50 Gew.-%, besonders bevorzugt von 30-50 Gew.-%, auf.

Falls zur Herstellung der Zellsuspension eine wässrige Lösung eingesetzt wird, dann kann diese insbesondere weitere Nahrungsmittelkomponenten - wie etwa Vitamine oder Salze - enthalten.

Weiterer Gegenstand der vorliegenden Erfindung ist auch ein Verfahren zur Herstellung eines Wertstoffs, bei welchem nach Durchführung des erfindungsgemäßen Zellaufschlusses unter Verwendung des Rotor-Stator-Systems anschließend eine teilweise oder vollständige Abtrennung der Zelltrümmer erfolgt, um einen teilweise oder vollständig gereinigten Wertstoff zu erhalten. Weiterer Gegenstand der vorliegenden Erfindung ist entsprechend auch der Wertstoff, der durch ein solches erfindungsgemäßes Verfahren erhältlich ist.

Bei dem Wertstoff handelt es sich entsprechend vorzugsweise um ein Lipid, vorzugsweise ein Öl, besonders bevorzugt um ein Öl, das polyungesättigte Fettsäuren enthält, wobei vorzugsweise mindestens 10 Gew.-%, insbesondere mindestens 20 Gew.-%, besonders bevorzugt 20 - 60 Gew.-%, vor allem 20 - 40 Gew.-%, der in der Zelle enthaltenden Lipide polyungesättigte Fettsäuren darstellen. Vorzugsweise werden hierbei mindestens 20, 30 oder 40 Gew.-%, insbesondere mindestens 50, 60 oder 70 Gew.-%, besonders bevorzugt mindestens 80, 90 oder 95 Gew.-% der Zelltrümmer abgetrennt.

Die Abtrennung der Zelltrümmer erfolgt erfindungsgemäß vorzugsweise mechanisch, beispielsweise durch Denkantieren, Filtration oder Zentrifugation.

Zur Abtrennung des Wertstoffs von den Zelltrümmern kann gegebenenfalls auch ein Lösungsmittel verwendet werden, in welchem sich der Wertstoff löst. Das Lösungsmittel kann nach der Abtrennung des Wertstoffs entsprechend wieder entfernt werden, beispielsweise durch Anlegen eines reduzierten Drucks. Alternativ kann die Abtrennung des Wertstoffs beispielsweise durch superkritische Flüssigextraktion erfolgen.

So kann die Abtrennung eines Öls beispielsweise dadurch erfolgen, dass die dem Fachmann bekannten Lösungsmittel, beispielsweise Chloroform, Ether, Hexan, Methylenchlorid oder Methanol, eingesetzt werden. Die Abtrennung des Öls kann beispielsweise auch dadurch erfolgen, dass ein anderes Öl zur Extraktion des erfindungsgemäßen Öls verwendet wird. Alternativ kann die Abtrennung des Öls natürlich auch ohne Verwendung eines Lösungsmittels auf rein mechanischem Wege erfolgen.

Das Öl kann anschließend einer chemischen oder physikalischen Raffinierung unterzogen werden. Die Raffinierung kann das Degummieren, das Bleichen, das Filtern, das Desodorieren und/oder das Polieren des Rohöls umfassen.

Die Isolierung gewünschter Öl-Bestandteile, insbesondere der omega-3-Fettsäuren, kann durch Hydrolyse der enthaltenen Lipide und anschließende fraktionierte Kristallisation, fraktionierte Destillation, Säulenchromatographie oder superkritische Flüssigfraktionierung erfolgen. Die Hydrolyse der Lipide kann hierbei durch basische, saure oder enzymatische Spaltung erfolgen. Nach der Spaltung der Lipide können die nicht-verseifbaren Komponenten durch Lösungsmittelextraktion - beispielsweise unter Verwendung von Ether, Hexan oder Chloroform - abgetrennt werden. Die verbliebene Lösung wird anschließend acidifiziert, so dass die freien Fettsäuren ebenfalls in ein Lösungsmittel aufgenommen werden können. Durch Kühlung können dann die nicht-polyungesättigen Fettsäuren aus dem Lösungsmittel ausgefällt und durch Filtration, Zentrifugation oder Dekantieren abgetrennt werden, während die polyungesättigen Fettsäuren in Lösung verbleiben.

Weiterer Gegenstand der vorliegenden Erfindung ist ein Verfahren zur Herstellung eines Nahrungs- oder Futtermittels, bei welchem die Zellen nach Durchführung des Zellaufschlusses mit anderen Nahrungs- oder Futtermittelinhaltsstoffen vermischt werden und anschließend zu dem Nahrungs- oder Futtermittel verarbeitet werden.

Weiterer Gegenstand der vorliegenden Erfindung ist auch ein Verfahren zur Herstellung eines Nahrungs- oder Futtermittels, bei welchem der teilweise oder vollständig gereinigte Wertstoff, insbesondere ein erfindungsgemäß isoliertes Lipid oder erfindungsgemäß isolierte omega-3-Fettsäuren, mit anderen Nahrungs- oder Futtermittelinhaltsstoffen vermischt wird und anschließend zu dem Nahrungs- oder Futtermittel verarbeitet wird.

Weiterer Gegenstand der vorliegenden Erfindung ist daher auch ein Nahrungs- oder Futtermittel, das durch erfindungsgemäße Verfahren erhältlich ist.

Die Verarbeitung des Gemisches aus aufgeschlossenen Zellen und weiteren Nahrungs- oder Futtermittelinhaltsstoffen erfolgt in einer bevorzugten Ausführungsform durch ein Extrusionsverfahren, um verkaufsfertige Portionen des Nahrungs- oder Futtermittels zu erhalten. Alternativ kann beispielsweise auch ein Pelletierverfahren eingesetzt werden.

Im Extrusionsverfahren wird vorzugsweise ein Schnecken- oder Doppelschneckenextruder eingesetzt. Das Extrusionsverfahren wird vorzugsweise bei einer Temperatur von 80 - 220° C, insbesondere 100 - 190 °C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Bei einer erfindungsgemäß bevorzugten Art des Extrusionsverfahrens umfasst das Verfahren einen Kompaktierungs- und einen Kompressionsschritt.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken.

Die weiteren Nahrungs- oder Futtermittelinhaltsstoffe werden vor dem Vermischen mit den aufgeschlossenen Zellen - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Ein erfindungsgemäß bevorzugtes Verfahren zur Herstellung eines Nahrungs- oder Futtermittels umfasst daher die folgenden Schritte:
a) Bereitstellung einer Zellsuspension, insbesondere von Zellen aus Thraustochytriales, mit einem Feuchtigkeitsgehalt von mindestens 30 Gew.-%, vorzugsweise 30 bis 90 Gew.-%, besonders bevorzugt 40 bis 80 Gew.-%, insbesondere 50 bis 75 Gew.-%, ausgehend von einer Zellmasse mit einem Feuchtigkeitsgehalt von weniger als 15 Gew.-%, vorzugsweise weniger als 10 Gew.-%, insbesondere 1 - 9 Gew.%, besonders bevorzugt weniger als 5 Gew.-%, insbesondere 1 - 4,5 Gew.-%;
b) Zellaufschluss unter Anwendung eines Energieeintrags von maximal 50 kWh pro Tonne Suspension, vorzugsweise von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, vorzugsweise unter Verwendung eines Rotor-Stator-Systems;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren. Erfindungsgemäße Verfahren zur Herstellung eines Nahrungs- oder Futtermittels zeichnen sich vorzugsweise dadurch aus, dass in keinem Verfahrensschritt, insbesondere auch nicht während der Extrusion, ein höherer Energieeintrag auf die Zellen bzw. aufgeschlossenen Zellen erfolgt als beim Zellaufschluss. Vorzugsweise ist der Energieeintrag in allen übrigen Verfahrensschritten, denen die Zellen bzw. aufgeschlossenen Zellen bzw. der enthaltene Wertstoff ausgesetzt sind, deutlich geringer als während des Zellaufschlusses. Der Energieeintrag beträgt hierbei in allen anderen Verfahrensschritten vorzugsweise maximal 80 %, insbesondere maximal 60 %, des Energieeintrags, der beim Zellaufschluss auf die Zellen erfolgt. Auf diese Weise wird sichergestellt, dass der enthaltene Wertstoff möglichst wenig geschädigt wird.

Die aufgeschlossenen Zellen machen vorzugsweise 0,5-20 Gew.-%, insbesondere 1-10 Gew.-%, vorzugsweise 2-8 Gew.-% des Nahrungs- oder Futtermittels bzw. der zur Herstellung des Nahrungs- oder Futtermittels eingesetzten Zusammensetzung aus.

Die durch ein zuvor beschriebenes erfindungsgemäßes Verfahren erhältliche Nahrungs- und Futtermittel zeichnen sich vorzugsweise durch eine sehr homogene Verteilung des Wertstoffs in dem erhältlichen Nahrungs- oder Futtermittel aus.

Weiterer Gegenstand der vorliegenden Erfindung ist daher ein Nahrungs- oder Futtermittel, das einen Wertstoff, insbesondere ein Lipid, vor allem PUFAs, enthält, wobei der Wertstoff sehr homogen in dem Nahrungs- oder Futtermittel verteilt ist. Der Wertstoff macht hierbei vorzugsweise 0,1 - 15 Gew.-%, besonders bevorzugt 0,2 - 8 Gew.-%, des Nahrungs- oder Futtermittels aus.

Die Homogenität der Verteilung kann hierbei auf folgende Weise bestimmt werden: Es werden n Proben mit einer Masse von jeweils x Gramm genommen. Anschließend wird jeweils die in den einzelnen Proben enthaltene Masse des Wertstoffes (y Gramm) bestimmt und der Mittelwert der Masse des in den n Proben enthaltenen Wertstoffes bestimmt (yₙ Gramm).

Erfindungsgemäße Nahrungs- oder Futtermittel zeichnen sich vorzugsweise dadurch aus, dass die Abweichung der enthaltenen Masse des Wertstoffes in den einzelnen Proben in Bezug auf den ermittelten Mittelwert der Masse des Werststoffes maximal 25 %, vorzugsweise maximal 20 oder 10 %, besonders bevorzugt maximal 5, 3 oder 2 % beträgt.

Dieses Merkmal ist hierbei vorzugsweise für mindestens eine der folgenden Vorgaben, vorzugsweise für alle folgenden Vorgaben, erfüllt: 20 oder 50 Stichproben des Nahrungs- oder Futtermittels mit einer Masse von jeweils 1,00 Gramm, 20 oder 50 Stichproben des Nahrungs- oder Futtermittels mit einer Masse von jeweils 500 Milligramm, 20 oder 50 Stichproben des Nahrungs- oder Futtermittels mit einer Masse von jeweils 100 Milligramm.

Weiterhin und/oder alternativ beträgt die Varianz der Verteilung des Wertstoffs in unterschiedlichen Teilportionen des Nahrungs- oder Futtermittels maximal 10 %, vorzugsweise maximal 5 %, insbesondere maximal 3 %.

Die Varianz wird hierbei vorzugsweise ebenfalls ermittelt für 20 oder 50 Stichproben des Nahrungs- oder Futtermittels mit einer Masse von jeweils 1,00 Gramm, 500 Milligramm oder 100 Milligramm, wobei das Merkmal der Varianz vorzugsweise für mindestens eine der Vorgaben, besonders bevorzugt für alle genannten Vorgaben, erfüllt ist.

Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Als proteinhaltige Komponente, die zusätzlich Fette enthält, können beispielsweise eingesetzt werden Fischmehl, Krillmehl, Muschelmehl, Kalmarmehl oder Schrimpsschalen. Als fetthaltige Komponente kann alternativ auch Fischöl eingesetzt werden. Als fetthaltige Komponente kann auch ein Pflanzenöl eingesetzt werden, insbesondere Öl aus Sojabohnen, Rapssamen, Sonnenblumenkernen und Flachssamen. Als kohlenhydrathaltige Komponente kann beispielsweise Weizenmehl, Sonnenblumenmehl, Sojablumenmehl oder Getreidegluten eingesetzt werden.

Der Gesamtgehalt an Öl in dem Futtermittel - inklusive des Öls aus den öl-haltigen Zellen - beträgt vorzugsweise 15-30 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

Weiterer Gegenstand der vorliegenden Erfindung ist entsprechend auch ein Verfahren zum Züchten von Tieren, insbesondere Flossenfischen oder Crustaceen, vorzugsweise Lachs, bei welchem ein erfindungsgemäßes Futtermittel eingesetzt wird. Weiterer Gegenstand der vorliegenden Erfindung ist weiterhin ein Tier, insbesondere Flossenfisch oder Schalentier, das durch ein derartiges erfindungsgemäßes Verfahren erhältlich ist.

## Patentansprüche

1. Verfahren zum Aufschluss von Zellen, die einen Wertstoff enthalten, **dadurch gekennzeichnet, dass** die Zellen vor dem Zellaufschluss aufgequollen werden.

2. Verfahren nach Anspruch 1, **dadurch gekennzeichnet, dass** es sich bei dem Wertstoff um ein Lipid handelt, wobei das Lipid vorzugsweise mindestens 20 Gew.-% der Zellmasse ausmacht und vorzugsweise polyungesättigte Fettsäuren enthält.

3. Verfahren nach Anspruch 1 oder 2, **dadurch gekennzeichnet, dass** das Aufquellen der Zellen dadurch erfolgt, dass ausgehend von einer Zellmasse mit einem Feuchtigkeitsgehalt von weniger als 15 Gew.-%, vorzugsweise weniger als 10 Gew.- %, insbesondere weniger als 5 Gew.-%, eine Zellsuspension mit einem Feuchtigkeitsgehalt von mindestens 30 Gew.-%, vorzugsweise 30 bis 90 Gew.-%, insbesondere 40 bis 80 Gew.-%, vor allem 50 bis 75 Gew.-%, eingestellt wird.

4. Verfahren nach einem der vorhergehenden Ansprüche, **dadurch gekennzeichnet, dass** zur Durchführung des Zellaufschlusses ein Rotor-Stator-System eingesetzt wird.

5. Verfahren nach einem der vorhergehenden Ansprüchen, **dadurch gekennzeichnet, dass** bei Durchführung des Zellaufschlusses ein Energieeintrag auf die Zellsuspension von maximal 50 kWh/t, vorzugsweise 0,1 - 50 kWh/t, besonders bevorzugt 0,5 - 40 kWh/t, insbesondere 1 - 30 kWh/t, vor allem 3 - 15 kWh/t, erfolgt.

6. Verfahren nach einem der vorhergehenden Ansprüche, **dadurch gekennzeichnet, dass** es sich bei den Zellen um mikrobielle Zellen, insbesondere um Labyrinthulomyceten, vorzugsweise der Familie der Thraustochytriaceae, handelt.

7. Verfahren zur Herstellung eines Wertstoffs, insbesondere eines Lipids, das vorzugsweise PUFAs enthält, umfassend ein Zellaufschlussverfahren nach einem der vorhergehenden Ansprüche, **dadurch gekennzeichnet, dass** nach Durchführung des Zellaufschlusses eine teilweise oder vollständige Abtrennung der Zelltrümmer erfolgt.

8. Verfahren nach einem der vorhergehenden Ansprüche, **dadurch gekennzeichnet, dass** die Zellen nach Durchführung des Zellaufschlusses - oder der (teilweise) isolierte Wertstoff- mit anderen Nahrungs- oder Futtermittelinhaltsstoffen vermischt werden und anschließend, vorzugsweise in einem Extrusionsverfahren, zu einem Nahrungs- oder Futtermittel verarbeitet werden.

9. Verfahren nach vorherigem Anspruch, **dadurch gekennzeichnet, dass** die aufgeschlossenen Zellen 0,5-20 Gew.-%, insbesondere 1-10 Gew.-%, vorzugsweise 2-8 Gew.-%, der im Extrusionsverfahren eingesetzten Zusammensetzung ausmachen und die anderen Nahrungs- oder Futtermittelinhaltsstoffe ausgewählt sind aus kohlenhydrathaltigen, proteinhaltigen, nukleinsäurehaltigen, lipidlöslichen und gegebenenfalls weiteren fetthaltigen Komponenten.

10. Verfahren zur Herstellung eines Nahrungs- oder Futtermittels umfassend die folgenden Schritte:
a) Bereitstellung einer Zellsuspension, insbesondere von Zellen aus Thraustochytriales, mit einem Feuchtigkeitsgehalt von mindestens 30 Gew.-%, vorzugsweise 30 bis 90 Gew.-%, besonders bevorzugt 40 bis 80 Gew.-%, ausgehend von einer Zellmasse mit einem Feuchtigkeitsgehalt von weniger als 15 Gew.-%, vorzugsweise weniger als 10 Gew.-%, insbesondere 1 - 9 Gew.-%;
b) Zellaufschluss unter Anwendung eines Energieeintrags von maximal 50 kWh pro Tonne Suspension, vorzugsweise von 0,1 - 50 kWh, insbesondere 0,8 - 35 kWh, vor allem 1 - 30 kWh, insbesondere 2 - 20 kWh oder 3 - 15 kWh, jeweils pro Tonne Suspension, vorzugsweise unter Verwendung eines Rotor-Stator-Systems;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

11. Wertstoff, erhältlich nach einem Verfahren gemäß Anspruch 7.

12. Nahrungs- oder Futtermittel, erhältlich nach einem Verfahren gemäß Anspruch 8, 9 oder 10.

13. Nahrungs- oder Futtermittel, das einen Wertstoff, vorzugsweise Lipide, insbesondere PUFAs enthält, **dadurch gekennzeichnet, dass** der Wertstoff homogen in dem Nahrungs- oder Futtermittel verteilt ist, **dadurch gekennzeichnet, dass** die Varianz der Verteilung des Wertstoffs in unterschiedlichen Teilportionen des Nahrungs- oder Futtermittels maximal 10 %, vorzugsweise maximal 5 %, insbesondere maximal 3 %, beträgt.

14. Verfahren zum Züchten von Tieren, insbesondere Flossenfischen oder Crustaceen, vorzugsweise Lachs, **dadurch gekennzeichnet, dass** ein Nahrungs- oder Futtermittel gemäß Anspruch 12 oder 13 eingesetzt wird.

15. Tier, insbesondere Flossenfisch oder Schalentier, erhältlich durch ein Verfahren gemäß Anspruch 14.
